# EUROPÄISCHE PATENTSCHRIFT

(11) **EP 2 167 913 B1**
(45) Veröffentlichungstag und Bekanntmachung des Hinweises auf die Patenterteilung: **25.05.2011**
(21) Anmeldenummer: 08786154.8
(22) Anmeldetag: 15.07.2008
(51) Int. Cl.: G01B 21/32, A61B 5/11

(54) **MESSVORRICHTUNG FÜR DIE MESSUNG EINER BIEGUNG UND EINER VERWINDUNG**
MEASURING DEVICE FOR MEASURING A BENDING MOTION AND A TORSION
DISPOSITIF DE MESURE CONÇU POUR MESURER UNE COURBURE ET UNE TORSION

(30) Priorität: 18.07.2007 DE 102007034264; 21.09.2007 DE 102007046384
(43) Veröffentlichungstag der Anmeldung: 31.03.2010
(73) Patentinhaber: Siemens Aktiengesellschaft, 80333 München (DE)
(72) Erfinder: BARBOUTIS, Grigorios, 10589 Berlin (DE); GOLDBECK, Dirk David, 81927 München (DE); HAPPEL, Tobias, 10553 Berlin (DE); KWIATEK, Andre Matthias, 12159 Berlin (DE); NERRETER, Stefan, 15754 Heidesee OT Blossin (DE)
(74) Vertreter: Wolff, Harry
(86) Internationale Anmeldenummer: PCT/EP2008/059252
(87) Internationale Veröffentlichungsnummer: WO 2009/010517

(56) Entgegenhaltungen:
- EP-A- 1 744 165
- WO-A-86/01588
- WO-A-89/11247
- WO-A-98/41815
- WO-A-2007/110300
- US-B1- 6 612 992

## Beschreibung

Die Erfindung betrifft eine Messvorrichtung, aufweisend ein Trägerband, auf dem parallel verlaufend ein Biegesensor befestigt ist, der eine Verbiegung des Trägerbandes nachvollzieht und dabei ein von der Biegung abhängiges erstes Messsignal erzeugt. Außerdem sind auf dem Trägerband Verwindungssensoren angebracht, mit denen ein von der Verwindung des Trägerbandes abhängiges zweites Messsignal erzeugt wird.

Eine Messvorrichtung der eingangs genannten Art ist beispielsweise in der EP 968 400 B1 beschrieben. Diese Messvorrichtung weist ein Trägerband auf, auf dem eine Vielzahl von Verwindungssensoren und Biegesensoren im Wechsel angeordnet sein können. Wird das Trägerband, welches selbst biegeschlaff ist, verbogen bzw. um seine Längsachse verwunden, so wird der lokale Verformungszustand durch die jeweiligen Sensoren aufgenommen. Hierdurch kann eine Raumkurve rekonstruiert werden, in der das verformte Trägerband verläuft. Hierbei steigt der mit der Berechnung der Raumkurve verbundene Aufwand einerseits mit der Dichte der Einzelsensoren auf dem Trägerband, andererseits mit der Länge des Trägerbandes. Gleichzeitig muss bei der Auswertung berücksichtigt werden, dass sich evtl. auftretende Messfehler addieren.

Der WO 98/41815 A1 ist zu entnehmen, dass Beschleunigungssensoren im Wechsel mit Biegungssensoren auf einem Sensorband angebracht werden können, um den räumlichen Verlauf des Sensorbandes zu bestimmen. Dafür werden die Beschleunigungssensoren entlang der Längsausdehnung des Sensorbandes in bestimmten Abständen zueinander angebracht, wobei sich aus der Auswertung der Signale der Beschleunigungssensoren und der Biegungssensoren eine Raumkurve des Bandes, ausgehend von einem bestimmten Bezugspunkt, errechnen lässt.

Gemäß der EP 1 744 165 A1 ist es außerdem bekannt, dass Biegungssensoren paarweise verwendet werden können, so das eine Beschleunigung in mehreren Achsen ermittelbar wird. Hierzu werden beispielsweise zwei einachsige Beschleunigungssensoren im rechten Winkel zueinander angeordnet.

Aus der nachveröffentlichten WO 2007/110300 A1 ist bekannt, dass eine Verwindung eines Sensorbandes auch mittels Biegesensoren ermittelt werden kann, welche in achtförmigen Schleifen auf dem Sensorband verlaufen. Hierzu müssen diese Biegesensoren, die als optische Sensorfasern ausgeführt sind, an dafür geeigneten Stellen mit einer biegesensitiven Zone mit einer erhöhten optischen Dämpfung versehen werden.

Die Aufgabe der Erfindung liegt darin, eine Messvorrichtung anzugeben, mit der die Biegung und die Verwindung eines Trägerbandes, auf dem die beteiligten Sensoren montiert sind, mit vergleichsweise geringem Aufwand vergleichsweise genau ermittelt werden kann.

Diese Aufgabe wird mit der eingangs angegebenen Messvorrichtung erfindungsgemäß dadurch gelöst, dass die Verwindungssensoren mit einem bestimmten Abstand in Längsrichtung des Trägerbandes zueinander angeordnet sind und dass zweite Messsignal von einer Veränderung der Winkelstellung der Verwindungssensoren bezüglich der Verwindungslinie abhängig ist. Wenn der Abstand der Verwindungssensoren in Längsrichtung des Trägerbandes bekannt ist, so kann von einer Veränderung der Winkelstellung der Verwindungssensoren bezüglich der Verwindungslinie direkt auf den Grad der Verwindung des Trägerbandes geschlossen werden. Damit ist vorteilhaft lediglich durch den Einsatz von zwei Verwindungssensoren eine Aussage über die Verwindung in dem durch den bestimmten Abstand definierten Bereich des Trägerbandes möglich.

Erfindungsgemäß ist vorgesehen, dass die Verwindungssensoren durch Paare von Beschleunigungssensoren gebildet sind, die mit einem bestimmten Abstand von der Verwindungslinie beidseitig der Verwindungslinie einander gegenüberliegend angeordnet sind. Durch diese Anordnung von Beschleunigungssensoren wird folgendes Messprinzip verwirklicht. Im Falle einer Verwindung des Trägerbandes um die Verwindungslinie herum erzeugen die Beschleunigungssensoren während der Beschleunigungs- bzw. der Bremsphase der Bewegung gegensätzliche Beschleunigungssignale (d. h. Signale mit unterschiedlichem Vorzeichen). Diese lassen auf eine Verwindung schließen, wobei durch eine Zeitüberwachung gleichzeitig die Geschwindigkeit der Beschleunigungssensoren und somit bei bekanntem Abstand derselben von der Verwindungslinie auch der Verwindungswinkel berechnet werden kann. Die paarweise Anordnung der Beschleunigungssensoren ist notwendig, damit eine Fehlinterpretation einer Biegung des Trägerbandes als Verwindung ausgeschlossen werden kann. In dem Fall einer Verbiegung des Trägerbandes senden die Paare von Beschleunigungssensoren nämlich Beschleunigungssignale mit gleichem Vorzeichen. Durch geeignete Interpretation der Sensorsignale bleiben jedoch gleichartige Sensorsignale der Paare von Beschleunigungssensoren unberücksichtigt. In dem Fall, in dem eine Verwindung des Sensorbandes gleichzeitig mit einer Biegung erfolgt, werden die entsprechenden Beschleunigungswerte überlagert. Jedoch ist auch in diesem Fall durch eine einfache Differenzbildung der Anteil der Beschleunigung aufgrund auf der Biegung herauszurechnen, so dass auf den Zustand der Verwindung geschlossen werden kann.

Zu bemerken ist, dass das Trägerband an sich lediglich zur Konfiguration der Biegesensoren und Verwindungssensoren zum Einsatz kommt. Dieses lässt sich jedoch auf einen Körper aufbringen, welcher hinsichtlich seiner Biegung bzw. Verwindung beobachtet werden soll. Denkbar ist beispielsweise eine Applikation, mit der die Bewegung des Rückens des menschlichen Körpers festgestellt bzw. überwacht werden soll. Das Trägerband lässt sich in diesem Falle auf dem Rücken des Probanten applizieren, wobei der Biegesensor die Biegung der Wirbelsäule nachformt und auch ihre Bewegung überwachen kann. Die Verwindungssensoren werden bei diesem Anwendungsfall dazu verwendet, um eine Drehung der Schultern gegenüber dem Becken festzustellen. Bei diesem Anwendungsfall und auch bei anderen denkbaren Anwendungsfällen ist es besonders vorteilhaft, wenn die Verwindungssensoren an den beiden Enden des Trägerbandes angebracht sind. Dies bedeutet bei der Applikation des Trägerbandes am Rücken, dass sich der eine Verwindungssensor in Höhe des Beckens und der andere Verwindungssensor in Höhe der Schulterblätter befindet. Hierdurch lässt sich der maximal mögliche Ausschlag der Verwindungssensoren bei einer Wirbelsäulendrehung ermitteln.

Unter der Verwindungslinie des Trägerbandes ist diejenige Raumkurve zu verstehen, welche das Trägerband bei einer Biegung und einer Verwindung im Raum beschreibt. Man kann sich die Verwindungslinie durch die Zentren aller Querschnitte des Trägerbandes verlaufend vorstellen. Verläuft das Trägerband gerade, also ohne eine Verbiegung, so ergibt sich für die Verwindungslinie eine gerade verlaufende Verwindungsachse, um die herum die Enden des Trägerbandes gegeneinander verdreht werden können. Eine solche Verwindung ist jedoch auch möglich, wenn das Trägerband gebogen ist. Man stelle sich beispielsweise einen sich bückenden, stehenden Menschen vor, der etwas aufheben möchte. Dieser wird durch eine Flexion der Wirbelsäule seinen Oberkörper nach vorne beugen, jedoch, während er seine Hand nach dem aufzuhebenden Gegenstand ausstreckt, auch die zur entsprechenden Hand gehörende Schulter weiter nach vorne schieben. Hierbei ergibt sich eine Biegung des Trägerbandes, der eine Verwindung überlagert ist.

Um Messfehler vorteilhaft zu verringern, kann gemäß einer besonderen Ausgestaltung der Erfindung auch vorgesehen werden, dass die gegenüberliegenden Beschleunigungssensoren eines Paares jeweils auf Ansatzstücken befestigt sind, die das Trägerband beidseitig verlängern, wobei die Ansatzstücke und das Trägerband im unverformten Zustand in einer Ebene liegen. Hierdurch wird nämlich erreicht, dass bei einer bestimmten Verwindung des Trägerbandes aufgrund des vergrößerten Abstandes der Beschleunigungssensoren von der Verwindungslinie eine größere Beschleunigung verzeichnet wird. Dies ermöglicht eine fein abgestimmte Auswertung der Sensorsignale, da diese betragsmäßig größer sind.

Eine spezielle Ausgestaltung der Erfindung erhält man, wenn das Trägerband einseitig mit einer Klebeschicht versehen ist. Durch die Klebeschicht lässt sich das Trägerband unproblematisch auf einen beliebigen Untergrund aufkleben, dessen Verbiegung und Verwindung überwacht werden soll. Insbesondere ist die Verwendung einer Klebeverbindung auch für die Überwachung des menschlichen Rückens von Vorteil, wobei zu beachten ist, dass ein hautverträglicher Klebstoff verwendet werden muss.

Weitere Einzelheiten der Erfindung werden im Folgenden anhand der Zeichnungen beschrieben. Gleiche oder sich entsprechende Zeichnungselemente werden in den einzelnen Figuren jeweils mit den gleichen Bezugszeichen versehen und nur in soweit mehrfach erläutert, wie sich Unterschiede zwischen den einzelnen Figuren ergeben. Es zeigen:
- Figur 1: die Applikation eines Ausführungsbeispiels der erfindungsgemäßen Messvorrichtung auf dem Rücken eines Probanten in perspektivischer Ansicht,
- Figur 2: eine Aufsicht auf die Messvorrichtung gemäß Figur 1,
- Figur 3: perspektivisch in verstärkter Form verschiedene Verformungszustände der Messvorrichtung gemäß Figur 2 und
- Figuren 4 und 5: das Beschleunigungsverhalten der Beschleuni- gungssensoren über der Zeit bei unterschiedli- chen Belastungszuständen der Messvorrichtung gemäß Figur 2.

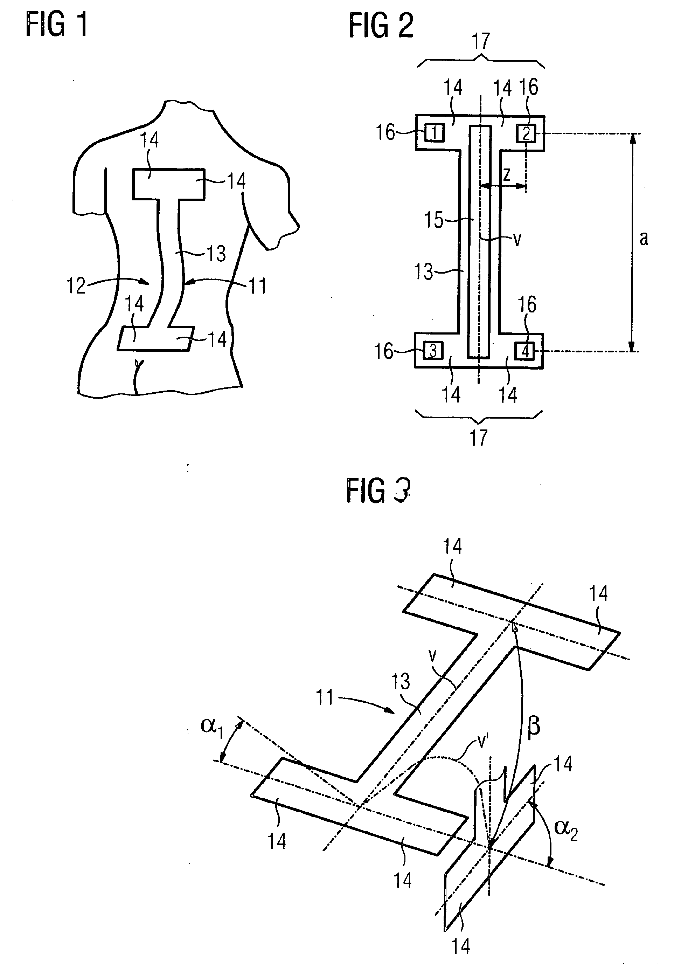

Der Figur 1 lässt sich entnehmen, wie eine erfindungsgemäße Messvorrichtung 11 auf dem Rücken 12 eines Probanden platziert werden kann. Die Messvorrichtung weist ein Trägerband 13 auf, an dessen Enden Ansatzstücke 14 vorgesehen sind.

Wie Figur 2 zu entnehmen ist, dient das Trägerband 13 zur Aufnahme eines Biegesensors 15. Auf den Ansatzstücken 14 sind weiterhin Beschleunigungssensoren 16 angeordnet, wobei jeweils ein Paar von Beschleunigungssensoren 16 am Ende des Trägerbandes 13 einen Verwindungssensor 17 ergeben. Hierbei ergibt sich zwischen den Verwindungssensoren ein Abstand a, der im Wesentlichen der Länge des Trägerbandes 13 entspricht. Weiterhin besitzen die Beschleunigungssensoren 16 jeweils einen Abstand z zu einer Verwindungslinie v, die im in Figur 2 dargestellten Zustand gerade der Mittelachse des Trägerbandes 13 entspricht.

Der Figur 1 ist zu entnehmen, dass die Beschleunigungssensoren 16 auf dem Rücken 12 jeweils im Bereich der Schulterblätter bzw. im Bereich des Beckens angeordnet sind. Hierdurch entsteht bei einer Verwindung der Wirbelsäule der für den gemäß Figur 1 dargestellten Messfall größtmögliche Verwindungswinkel zwischen den Ansatzstücken 14 der beiden Enden des Trägerbandes 13.

Die Messvorrichtung gemäß Figur 2 ist in Figur 3 perspektivisch dargestellt. Die Darstellung zeigt die Messvorrichtung im unverformten Zustand. Zu erkennen ist auch die Verwindungslinie v, die im unverformten Zustand gerade verläuft. Der Winkel α₁ zeigt, wie eine Verwindung des ansonsten unverformten Trägerbandes 13 der Messvorrichtung 11 aussehen könnte. Hierbei verändert sich die Verwindungslinie v nicht - sie bildet vielmehr eine Drehachse, um die das Trägerband 13 verwunden wird.

Weiterhin ist in Figur 3 ein komplexerer Verformungszustand dargestellt. Dieser beinhaltet eine Verbiegung des Trägerbandes 13 um einen Biegewinkel β = 90° und eine gleichzeitige Verwindung des Trägerbandes um α₂ = 90°. In diesem Falle ergibt sich eine gekrümmte Verwindungslinie v', wobei durch geeignete Auswertung der Sensorsignale der Anteil der Biegung und der Anteil der Verwindung unabhängig voneinander ermittelt werden kann.

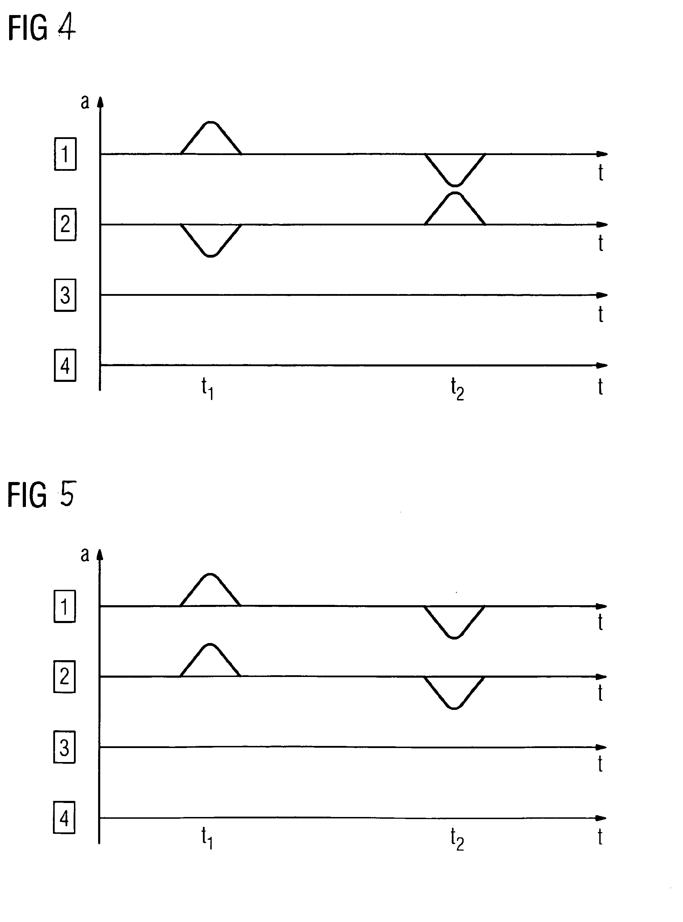

In Figur 4 ist exemplarisch dargestellt, wie verschiedene Bewegungen des Probanden gemäß Figur 1 durch die Beschleunigunssensoren aufgenommen werden können. Figur 4 stellt den Fall dar, dass sich der Proband beispielsweise umdreht, wobei die Wirbelsäule eine Torsion erfährt. Zu Beginn dieser Drehbewegung (Zeitpunkt t₁) erfahren die Beschleunigungssensoren 1 und 2, welche zwischen den Schulterblättern angeordnet sind (vgl. Figur 2) entgegengesetzte Beschleunigungen. Während sich der Oberkörper mit konstanter Geschwindigkeit dreht, ist an den Beschleunigungssensoren kein Signal zu messen. Zum Zeitpunkt t₂ ist die Bewegung des Umdrehens durch den Probanden abgeschlossen, wobei der sich drehende Oberkörper abgebremst werden muss. Hierbei entstehen an den Beschleunigungssensoren wieder entgegengesetzte Sensorsignale, die bezüglich der Sensorsignale zum Zeitpunkt t₁ jeweils das entgegengesetzte Vorzeichen haben.

In Figur 5 ist dargestellt, welche Signale die Beschleunigungssensoren senden, wenn sich der Proband bückt. Dabei bleiben die Beschleunigungssensoren 3 und 4 ähnlich wie beim Fall des Umdrehens gemäß Figur 4 weitgehend ruhig, weswegen keine Sensorsignale ermittelt werden. Anders die zwischen den Schulterblättern befindlichen Beschleunigungssensoren 1 und 2. Diese werden beim Nachvornebücken zunächst beschleunigt, und zwar diesmal in die gleiche Richtung, was gleichgerichtete Sensorsignale erzeugt (Zeitpunkt t₁). Während des Bückens mit konstanter Geschwindigkeit senden die Sensoren analog zum Fall gemäß Figur 4 kein Signal und sobald der Oberkörper wieder abgebremst wird, werden gleichgerichtete Sensorsignale (Zeitpunkt t₂) jedoch mit entgegensetztem Vorzeichen im Vergleich zur Beschleunigungsphase (Zeitpunkt t₁) erzeugt.

## Patentansprüche

1. Messvorrichtung, aufweisend ein Trägerband (13),
- auf dem parallel verlaufend ein Biegesensor (15) befestigt ist, der eine Verbiegung des Trägerbandes nachvollzieht und dabei ein von der Biegung abhängiges erstes Messsignal erzeugt und
- auf dem Verwindungssensoren (17) angebracht sind, mit denen ein von der Verwindung des Trägerbandes (13) abhängiges zweites Messsignal erzeugt wird,
wobei
- die Verwindungssensoren (17) mit einem bestimmten Abstand (a) in Längsrichtung des Trägerbandes (13) zueinander angeordnet sind und das zweite Messsignal von einer Veränderung der Winkelstellung der Verwindungssensoren bezüglich der Verwindungslinie (v) abhängig ist, **dadurch gekennzeichnet, dass** die Verwindungssensoren durch Paare von Beschleunigungssensoren (16) gebildet sind, und dass
die Beschleunigungssensoren (16) mit einem bestimmten Abstand (z) von der Verwindungslinie (v) beidseitig der Verwindungslinie (v) einander gegenüberliegend angeordnet sind und im Falle einer Verwindung des Trägerbandes (13) um die Verwindungslinie (v) herum Bescheunigungssignale mit unterschiedlichen Vorzeichen erzeugen.

2. Messvorrichtung nach Anspruch 1,
**dadurch gekennzeichnet,**
**dass** die Verwindungssensoren (17) an den beiden Enden des Trägerbandes (13) angebracht sind.

3. Messvorrichtung nach Anspruch 1 oder 2,
**dadurch gekennzeichnet,**
**dass** die gegenüberliegenden Beschleunigungssensoren (16) eines Paares jeweils auf Ansatzstücken (14) befestigt sind, die das Trägerband (13) beidseitig verlängern, wobei die Ansatzstücke (14) und das Trägerband (13) im unverformten Zustand in einer Ebene liegen.

4. Messvorrichtung nach einem der voranstehenden Ansprüche,
**dadurch gekennzeichnet,**
**dass** das Trägerband (13) einseitig mit einer Klebeschicht versehen ist.

## Claims

1. Measuring device having a carrier strip (13)
- to which a bending sensor (15) is fastened such that it runs in a parallel manner, which bending sensor understands bending of the carrier strip and in the process generates a first measurement signal dependent on the bending, and
- to which torsion sensors (17) are fitted, which torsion sensors are used to generate a second measurement signal dependent on the torsion of the carrier strip (13),
- the torsion sensors (17) being arranged at a particular distance (a) from one another in the longitudinal direction of the carrier strip (13), and the second measurement signal being dependent on a change in the angular position of the torsion sensors with respect to the torsion line (v), **characterized in that**
the torsion sensors are formed by pairs of acceleration sensors (16), and **in that**
the acceleration sensors (16) are arranged opposite one another at a particular distance (z) from the torsion line (v) on both sides of the torsion line (v) and generate acceleration signals with different signs in the event of torsion of the carrier strip (13) around the torsion line (v).

2. Measuring device according to Claim 1,
**characterized in that**
the torsion sensors (17) are fitted to the two ends of the carrier strip (13).

3. Measuring device according to Claim 1 or 2,
**characterized in that**
the opposite acceleration sensors (16) in a pair are each fastened to attachment pieces (14) which extend the carrier strip (13) on both sides, the attachment pieces (14) and the carrier strip (13) lying in one plane in the undeformed state.

4. Measuring device according to one of the preceding claims,
**characterized in that**
the carrier strip (13) is provided with an adhesive layer on one side.

## Revendications

1. Dispositif de mesure, comportant une bande (13) de support,
- sur laquelle est fixée un capteur (15) de flexion, qui s'étend parallèlement et qui reproduit une flexion de la bande de support et produit ainsi un premier signal de mesure qui dépend de la flexion et
- sur lequel sont mis des capteurs (17) de torsion, par lesquels un deuxième signal de mesure qui dépend de la torsion de la bande ( 13 ) de support est produit,
dans lequel
- les capteurs ( 17 ) de torsion sont disposés les uns par rapport aux autres à une distance ( a ) déterminée dans la direction longitudinale de la bande ( 13 ) de support et le deuxième signal de mesure dépend d'une modification de la position angulaire des capteurs de torsion par rapport à la ligne ( v ) de torsion,
**caractérisé en ce que** les capteurs de torsion sont formés par des paires de capteurs ( 16 ) d'accélération et **en ce que** les capteurs ( 16 ) d'accélération sont disposés en étant opposés les uns aux autres à une distance ( z ) déterminée de la ligne ( v ) de torsion des deux côtés de la ligne ( v ) de torsion et, dans le cas d'une torsion de la bande ( 13 ) de support autour de la ligne ( v ) de torsion, produisent des signaux d'accélération de signe différent.

2. Dispositif de mesure suivant la revendication 1,
**caractérisé**
**en ce que** les capteurs ( 17 ) de torsion sont mis aux deux extrémités de la bande ( 13 ) de support.

3. Dispositif de mesure suivant la revendication 1 ou 2,
**caractérisé**
**en ce que** les capteurs ( 16 ) d'accélération opposés d'une paire sont fixés respectivement sur des pièces ( 14 ) de prolongement, qui prolongent la bande ( 13 ) de support des deux côtés, les pièces ( 14 ) de prolongement et la bande ( 13 ) de support se trouvant dans un plan à l'état non déformé.

4. Dispositif de mesure suivant l'une des revendications précédentes,
**caractérisé**
**en ce que** la bande ( 13 ) de support est pourvue d'un côté d'une couche de colle.
